# EUROPEAN PATENT APPLICATION

(11) **EP 2 308 378 A2**
(43) Date of publication of application: **13.04.2011**
(21) Application number: 10251749.7
(22) Date of filing: 06.10.2010
(51) Int. Cl.: A61B 17/00, A61B 17/04

(54) **Wound closure device including releasable barbs**

(30) Priority: 07.10.2009 US 249294 P; 01.10.2010 US 895977
(71) Applicant: Tyco Healthcare Group LP, New Haven, CT 06511 (US)
(72) Inventor: Smith, Robert C., Middletown, CT 06457 (US)
(74) Representative: Soames, Candida Jane

(57) **Abstract**

A suturing device (10) includes an elongated tubular member (100) defining a longitudinal axis ("A"). The elongated tubular member (100) has a proximal portion (104) and a distal portion (102). A head assembly (200) extends from the distal portion (102) of the elongated tubular member (100) and is disposed about the longitudinal axis ("A"). The head assembly (200) includes one or more rotatable barb holders (220,240) coupled thereto. The rotatable barb holders (220,240) are configured to releasably retain a barb (230,250) thereon. The rotatable barb holders (220,240) are rotatable between a first position, wherein the barb (230,250) is positioned adjacent the elongated tubular member (100), and a second position, wherein the barb (230,250) is radially spaced from the elongated tubular member (100).

## Description

### CROSS-REFERENCE TO RELATED APPLICATION

The present application claims the benefit of and priority to U.S. Provisional Application Serial No. 61/249,294 filed on October 7, 2009, the entire contents of which are incorporated by reference herein.

### BACKGROUND

### Technical Field

The present disclosure relates to a wound closure device and, more particularly, to a wound closure device including releasable barbs for suturing a wound.

### Background of Related Art

Puncture wounds, wounds that pierce through tissue, may result from trauma or may be intentionally created in order to provide access to a body cavity during surgical procedures. During endoscopic surgical procedures, for example, a trocar device is utilized to puncture the peritoneum to provide an access port by way of a cannula through the abdominal wall. Generally, a trocar and/or cannula is placed through the abdominal wall for introduction of surgical instrumentation which is necessary to carry out the surgical procedure. In this manner, the surgeon may introduce a surgical instrument such as a grasper, scissor, ciip applier, stapler or any other surgical instrument which may be necessary during the particular surgical procedure. Once the procedure is complete, it is necessary to suture the wound.

Conventional instruments for closing puncture wounds generally include a shaft that can be extended into the body through either the puncture wound itself (in the case of a puncture caused by trauma) or through a cannula (in the case of a puncture created to access a surgical site). Suture retaining needles are then deployed from the shaft into tissue. Unfortunately, the mechanisms used for deploying the needles are often cumbersome and may make the extension and/or retraction of the suturing device difficult.

In the prior art, U.S. Patent No. 5,470,330 discloses a suturing instrument for closing trocar puncture wounds. The suturing instrument includes a pair of needle carriers having needles releasably retained thereon. The needle carriers are translatable from a retracted position to a deployed position via a rack and pinion mechanism to urge the needles into tissue. U.S. Patent No. 6,911,034 discloses a suturing apparatus including one or more arms having a suture mounting portion and one or more needles. Each arm is extendable from the suturing apparatus to penetrate tissue, while each needle is moveable from a first position adjacent the suturing apparatus to a second position adjacent an arm to capture the suture mounted on the suture mounting portion and draw the suture back toward the suturing apparatus. U.S. Patent No. 7,160,309 discloses a suturing device including a plurality of extendable projections configured to retain sutures thereon. Needles may then be passed through tissue to retrieve the sutures from the extendable protection. U.S. Patent No. 7,235,087 discloses an articulating suturing device including a shaft having an articulated foot disposed at a distal end thereof. The foot includes suture attachment cuffs that lockingly engage needles such that the cuffs can be withdrawn upon withdrawal of the needles. U.S. Patent No. 7,449,024 discloses a suturing device having at least two arms that are extendable from the shaft of the suturing device. The arms are rotated about a pivot to extend from the shaft. Needles may then be inserted into engagement with the arms to retrieve a suture from the arms. U.S. Patent Application Publication No. 2005/0119670 discloses a closure device including a pair of needles retaining a suture thereon and disposed at a distal end of a cannula. The needles are moveable from a first position, wherein the needles are disposed within the cannula for insertion, to a second position, wherein the needles are extended to penetrate into tissue, to a third position, wherein the needles are detached from the distal end of the cannula and are retained within the cannula, leaving the suture disposed through tissue. U.S. Patent Application Publication No. 2005/0228405 discloses a suturing apparatus including a rotatary portion and a pair of needles configured to retain a suture thereon. The needles are selectively advanced through tissue and into the rotary portion to retain the suture within the rotary portion. U.S. Patent Application Publication No. 2006/0052801 discloses a suturing instrument including a pair of suture passers each configured to retain a suture carrier tip thereon. The suture passers are deployed from the instrument and through tissue to engage the suture carrier tips with the instrument. The suture passers may then be returned, leaving the suture disposed through tissue.

### SUMMARY

In accordance with one embodiment of the present disclosure, a suturing device is provided. The suturing device includes an elongated tubular member defining a longitudinal axis and having a proximal portion and a distal portion. A head assembly extends from the distal portion of the elongated tubular member and is positioned about the longitudinal axis. The head assembly includes one or more rotatable barb holders coupled thereto. Each rotatable barb holder is configured to releasably retain a barb thereon. The rotatable barb holder(s) is rotatable between a first position, wherein the barb is positioned adjacent the elongated tubular member, and a second position, wherein the barb is radially spaced from the elongated tubular member.

In one embodiment, the suturing device further includes a handle assembly extending from the proximal portion of the elongated tubular member. A portion, or the entire handle assembly is rotatable with respect to the elongated tubular member to rotate the rotatable barb holder(s) between the first and second positions.

In another embodiment, the head assembly includes two rotatable barb holders. The two barb holders are diametrically opposed with respect to the longitudinal axis when in the second position.

In yet another embodiment, the elongated tubular member is configured to house a length of suture therethrough. Additionally, or alternatively, the head assembly may be configured to allow passage of the length of suture therethrough. Further, the length of suture may extend from the head assembly to engage the barbs disposed on the rotatable barb holders.

In still another embodiment, the barbs are formed from a bio-absorbable material. Accordingly, the barbs need not be removed after the suturing procedure is complete.

In still yet another embodiment, the barbs are disposed within an outer radius of the elongated tubular member when in the first position. Such a configuration facilitates passage of the suturing device through an opening in tissue.

Another embodiment of a suturing device provided in accordance with the present disclosure includes an elongated tubular member defining a longitudinal axis and having a proximal portion and a distal portion. The suturing device is configured to house a length of suture therethrough. A head assembly extends from the distal portion of the elongated tubular member is positioned about the longitudinal axis. A portion, or the entire head assembly is configured to allow passage of the length of suture therethrough. The head assembly further includes one or more rotatable barb holders extending therefrom. The rotatable barb holders are configured for retaining a barb thereon and are rotatable between a first position and a second position. In the first position, the barbs are disposed adjacent the elongated tubular member. In the second position, the barbs are radially spaced from the elongated tubular member.

In one embodiment, the suturing device includes a rotatable collar disposed on the proximal portion of the elongated tubular member. The rotatable collar is configured for rotating the rotatable barb holder(s) between the first position and the second position.

In one embodiment, each barb holder is adapted to releasably accommodate one or more barbs thereon and/or may be configured to retain the barb(s) thereon in a generally proximally-facing orientation. The suturing device may further be configured according to any of the above embodiments.

A method for suturing is also provided in accordance with the present disclosure. The method includes providing a suturing device according to any of the above embodiments. Next, with the barb holders in the first position, the suturing device is translated proximally through an opening in tissue to position the barbs on an interior side of tissue. The rotatable barb holders are then moved to the second position and the suturing device is translated with respect to tissue to engage the barbs within tissue. The barbs may then be released from the barb holders such that the suturing device may be returned to the first position and withdrawn from the opening in tissue.

In one embodiment, the method further includes tying off the length of suture to close the opening in tissue.

In another embodiment, the suturing device is translated proximally with respect to tissue to engage the barbs within tissue on either side of the opening in tissue. To release the barbs from the rotatable barb holders, the suturing device may be translated distally once the barbs have been engaged within tissue.

### BRIEF DESCRIPTION OF THE DRAWINGS

Various embodiments of the subject instrument are described herein with reference to the drawings wherein:

Fig. 1A is a side perspective view of a suturing device in accordance with the present disclosure including a pair of barb holders shown in an open position;

Fig. 1B is a side, perspective view of the suturing device of Fig. 1A with the barb holders disposed in a closed position;

Fig. 2 is a perspective view of a handle assembly of the suturing device of Fig. 1A with a portion of a housing removed to shown the internal components therein;

Fig. 3 is an enlarged view of a head assembly of the suturing device of Fig. 1A;

Fig. 4 is an exploded view of the suturing device of Fig. 1A;

Fig. 5 is a side, cross-sectional view of the suturing device of Fig. 1A with the barb holders in the closed position;

Fig. 6 is a transverse, cross-sectional view of the suturing device of Fig. 5 taken along section line 6-6;

Fig. 7 is a transverse, cross-sectional view of the suturing device of Fig. 5 taken along section line 7-7;

Fig. 8 is a side, cross-sectional view of the suturing device of Fig. 1A with the barb holders disposed in the open position;

Fig. 9 is a transverse, cross-sectional view of the suturing device of Fig. 8 taken along section line 9-9;

Fig. 10 is a transverse, cross-sectional view of the suturing device of Fig. 8 taken along section line 10-10;

Fig. 11 is a perspective view of the suturing device of Fig. 1A being inserted into tissue;

Fig. 12 is a side view of the distal portion of the suturing device of Fig. 1A disposed through an opening in tissue with the barb holders in the open position;

Fig. 13 is a side view of the distal portion of the suturing device of Fig. 1A disposed through an opening in tissue with the instrument translated proximally to engage the barbs with tissue;

Fig. 14 is a side view of the distal portion of the suturing device of Fig. 1A disposed through an opening in tissue with the instrument translated distally to release the barbs from the barb holders;

Fig. 15 is a side view of the distal portion of the suturing device of Fig. 1A disposed through an opening in tissue with the barb holders moved to the closed position once the barbs have been released; and

Fig. 16 is a side view of the barbs disposed within tissue once the barbs have been released from the instrument, each barb including a portion of suture retained therethrough.

### DETAILED DESCRIPTION

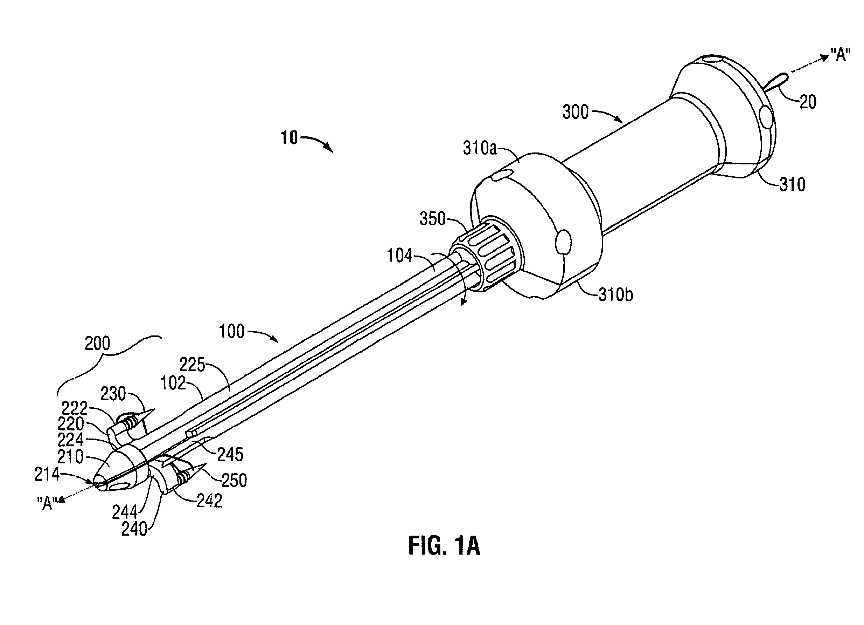

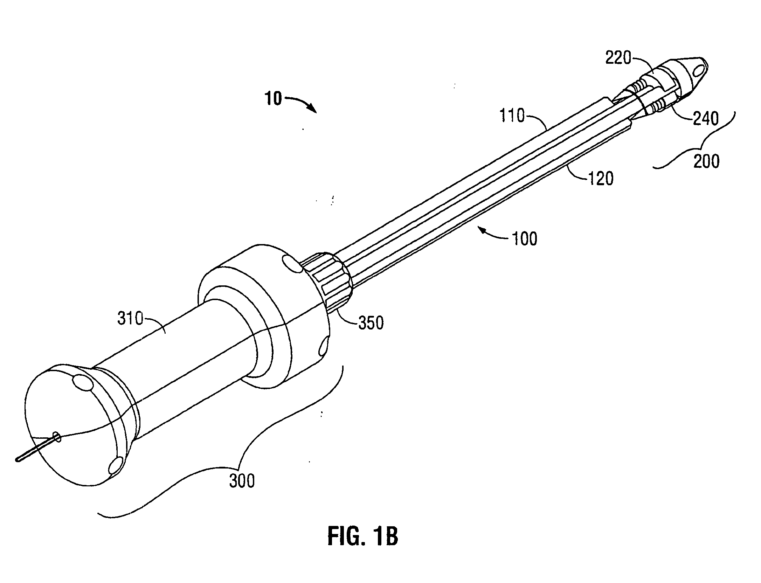

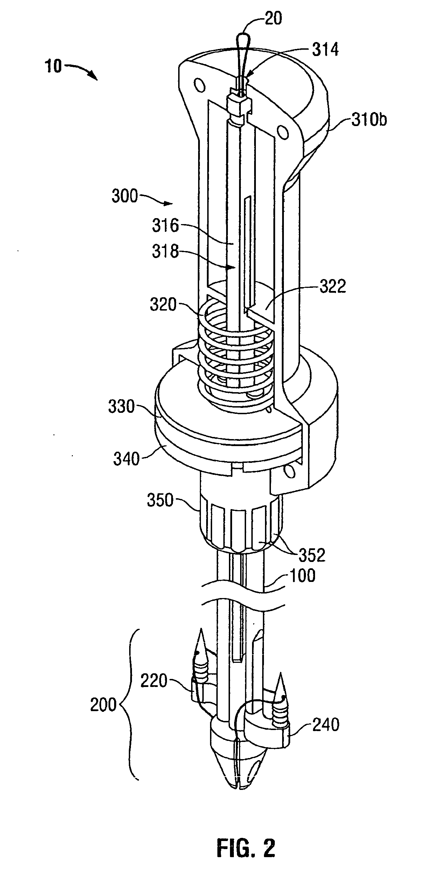

Turning now to Figs. 1A and 1B, suturing device 10 is shown including an elongated tubular portion 100 having a head assembly 200 disposed at a distal end 102 thereof and a handle assembly 300 disposed at a proximal end 104 thereof. Handle assembly 300 is formed from two housing parts 310a and 310b which, together, form housing 310 for housing the interior components of handle assembly 300. Longitudinal axis "A" is defined through the suturing device 10. Suture 20 is disposed through a lumen 318 (Fig. 2) which extends from aperture 314 (Fig. 2) defined within housing 310 of handle assembly 300 to communicate with elongated tubular portion 100, such that suture 20 may be disposed through elongated tubular portion 100 to head assembly 200 and out through aperture 214 defined within cap portion 210 of head assembly 200. Collar 350 is disposed at proximal end 104 of elongated tubular member 100 and is configured to rotate about longitudinal axis "A," to rotate barb holders 220 and 240 of head assembly 200 between the open and closed positions, as will be described in greater detail below. As shown in Fig. 1A, barb holders 220 and 240 having barbs 230 and 250, respectively, disposed thereon, are in the open position.

Referring now to Fig. 1B, suturing device 10 is shown wherein the barb holders 220 and 240 are disposed in the closed position. As shown in Figs. 1A and 1B, barb holder 220 retains barb 230 at a free end 222 thereof. A rod 225 extends along elongated tubular portion 100 engaging fixed end 224 of barb holder 220 at a first end thereof and engaging collar 350 at a second end thereof, thus coupling barb holder 220 to collar 350. Similarly, barb holder 240 retains a barb 250 on a free end 242 thereof and engages rod 245 at a fixed end 244 thereof. Rod 245 extends along elongated tubular portion 100 to engage collar 350 such that barb holder 240 is coupled to collar 350. Divider walls 110 and 120 are disposed along and extend radially outward from elongated tubular portion 100 to separate rod 225 from rod 245. The transitioning of barb holders 220 and 240 between the open and closed positions, including description of the inner workings of collar 350, will be described in greater detail below.

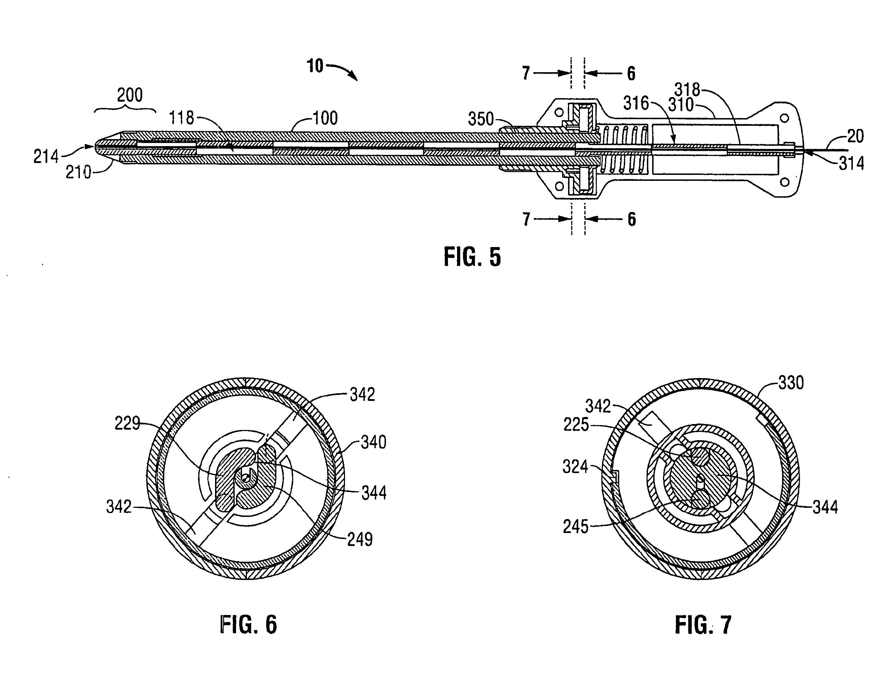

With reference now to Fig. 2, part 310a of housing 310 has been removed to show the internal components of handle assembly 300. Handle assembly 300 defines a conduit 316 extending through housing 310 of handle assembly 300. The conduit 316 extends from aperture 314 defined within housing 310 and defines lumen 318 therein. Lumen 318 of conduit 316 is in communication with lumen 118 (Fig. 5) defined through elongated tubular portion 100 such that suture 20 may be disposed through an entire length of the suturing device 10, e.g., entering the instrument through aperture 314 of housing 310 and exiting the instrument through aperture 214 (Fig. 1A) defined within cap portion 210 of head assembly 200.

With continued reference to Fig. 2, handle assembly 300 further includes a spring 320 surrounding conduit 316 and positioned between fixed wall portion 322 of housing 310 and disc 330. Spring 320 biases disc 330 distally. Collar 350 includes disc portion 340 disposed adjacent to and on a distal side of disc 330. Collar 350 may further include a plurality of notches 352 disposed around an outer surface thereof to facilitate hand-rotation of collar 350.

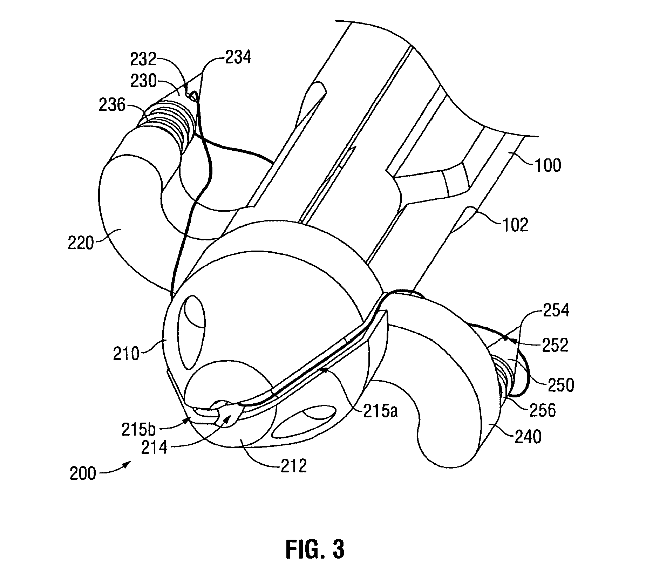

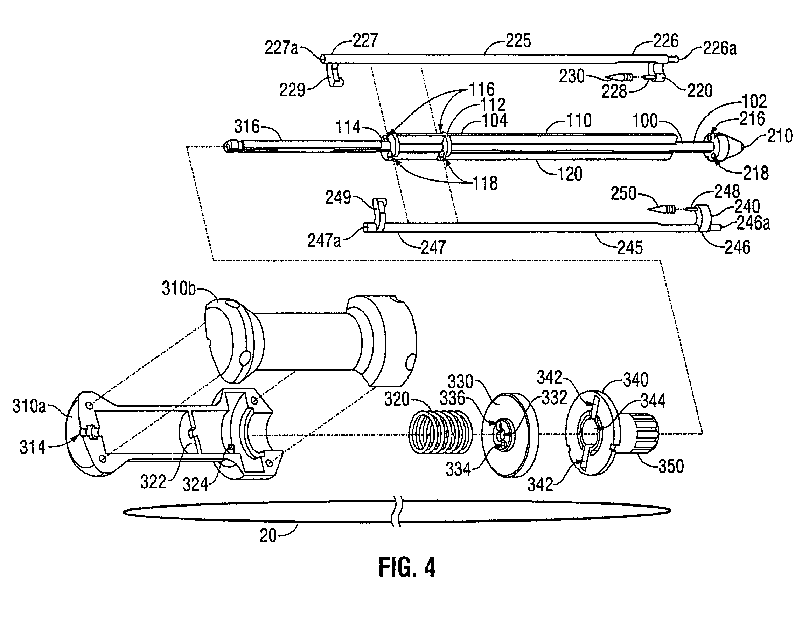

Referring now to Fig. 3, head assembly 200 is shown disposed at distal end 102 of elongated tubular member 100 and includes cap portion 210. Cap portion 210 may be generally conically shaped to facilitate the insertion of suturing device 10 through an opening in tissue and/or may also include a blunt tip portion to help avoid damaging tissue further or puncturing the cannula upon insertion into an opening in tissue or through a cannula, respectively. Aperture 214 defined within cap portion 210 of head assembly 200 communicates with lumen 118 (Fig. 5) defined through elongated tubular member 100, as mentioned above, such that suture 20 may enter suturing device 10 via aperture 314 defined within housing 310 (Fig. 2), travel through lumen 318 of conduit 316 into lumen 118 of tubular body portion 100 and exit via aperture 214 defined within cap portion 210 of head portion 200. Channels 215a and 215b (collectively channels 215) are defined along the outer surface of cap portion 210 and are configured to retain suture 20 therein, reducing the likelihood that suture 20 will catch, or snag on tissue, or other instrumentation during insertion of suturing device 10. As shown in Fig. 3, suture 20 extends from aperture 214, through channels 215, to barbs 230 and 250, that are releasably disposed on barb holders 220 and 240, respectively. Barbs 230 and 250 include apertures 232 and 252, respectively, defined therethrough for retaining a portion of suture 20. Suture 20 may be a loop such that, as shown in Fig. 3, suture 20 exits aperture 214, extends through channel 215a and through aperture 252 of barb 250, then through aperture 232 of barb 230 before returning up through channel 215b and back through aperture 214 of cap portion 210 (see also Fig. 4).

With continued reference to Fig. 3, barb 230 includes a tip portion 234 and a ribbed portion 236. Barb 250 is similar to barb 230 and includes a tip portion 254 and a ribbed portion 256. The tip portions 234 and 254 of barbs 230 and 250, respectively, are conically-shaped and may include sharp points such that barbs 230 and 250 to facilitate entry into and passage through tissue. The ribbed portions 236 and 256 of barbs 230 and 250, respectively, provide traction for the barbs 230, 250 once disposed through tissue such that barbs 230 and 250 may be securely anchored within tissue. Additionally, ribbed portions 236 and 256 may include distally-angled flanges (not explicitly shown) or other features to help inhibit barbs 230 and 250 from sliding distally out of tissue.

Referring now to Fig. 4, the suturing device 10 is shown with parts separated. As mentioned above, elongated tubular member 100 includes a pair of diametrically-opposed divider walls 110 and 120 disposed along a length thereof. A pair of spaced apart discs 112, 114 is disposed about elongated tubular member 100 at a proximal end 104 thereof. Discs 112 and 114 each include a pair of notches 116 and 118, respectively, defined therein. More specifically, notches 116 are defined within discs 112 and 114 on one side of elongated tubular member 100 as defined by divider walls 110 and 120, while notches 118 are defined within discs 112 and 114 on the opposite side of elongated tubular member 100. Notches 116 are configured and dimensioned to secure a portion of rod 225 therein and notches 118 are similarly configured and dimensioned to secure a portion of rod 245 therein. Rod 225, which is securable to elongated tubular member 100 by notches 116, includes distal knob portion 226a dimensioned to engage aperture 216 of cap member 210 of head assembly 200. Proximal knob portion 227a of rod 225 is dimensioned to engage aperture 336 of disc 330 at a proximal end of rod 225. Similarly, rod 245 includes distal knob portion 246a configured to engage aperture 218 of cap member 210 and proximal knob portion 247a, that is configured to engage aperture 334 of disc 330. As can be appreciated, the engagement of distal knobs 226a and 246a with apertures 216 and 218, respectively, and the engagement of proximal knobs 227a and 247a with apertures 336 and 334, respectively, secures rods 225 and 245 in place and allow rods 225 and 245 to be rotated, as will be discussed in greater detail below, with respect to elongated tubular member 100. Barb holder 220, which is engaged to rod 225, and barb holder 240, which is engaged to rod 245 are similarly rotated upon the rotation of rods 225, 245 to rotate barbs 230 and 250, respectively, disposed thereon from the closed position to the open position.

With continued reference to Fig. 4, rods 225 and 245 further include proximal flanges 229 and 249, respectively, extending perpendicularly from the respective rods 225 and 245. When assembled, proximal portions 227 and 247 of rods 225 and 245, respectively, are disposed through central aperture 344 of collar 350 such that proximal knobs 227a and 247a engage apertures 336 and 334 of disc 330, respectively. Cam slots 342 extend diametrically from central aperture 344 of collar 350. When barb holders 220 and 240 are disposed in the closed position, proximal flanges 229 and 249 are disposed within central aperture 344 of collar 350. When collar 350 is rotated, proximal flanges 229 and 249 are rotated to extend through cam slots 342 of rotating member 350. At the same time, the rotation of proximal flanges 229 and 249 from central aperture 344 through cam slots 342 rotates rods 225 and 245, thereby rotating barb holders 220 and 240 to the open position.

Barb holders 220 and 240 each include a barb post 228 and 248, respectively for retaining the barbs 230, 250 thereon. Apertures (not explicitly shown) may be defined within barbs 230 and 250 such that posts 228 and 248 may engage barbs 230 and 250 in a male-female connection to retain barbs 230 and 250 thereon. When barb holders 220 and 240 are in the closed position, barbs 230 and 250 are inhibited from sliding off of posts 228 and 248, respectively, due to positioning of divider walls 110 and 120. Similarly, cap member 210 protects barbs 230 and 250 while in the closed position, e.g., during insertion into an opening in tissue or a cannula. It is envisioned that barb holder 220, 240 may be biased toward the closed position or, alternatively, toward the open position. Locking mechanisms (not shown) are also contemplated for fixing barb holders 220, 240 in the open and/or closed positions, to inhibit inadvertent deployment/retraction of barb holder 220, 240.

Turning now to Figs. 5-7, suturing device 10 is shown with barb holders 220 and 240 disposed in the closed position. When in the closed position, barbs 230 and 250 (Fig. 4) are disposed within the dimensions of cap member 210 and elongated tubular member 100, such that neither barb holders 220 and 240 no barbs 230 and 250 protrude radially from elongated tubular member 100. Such a configuration protects barbs 230, 250 from being disengaged from barb holders 220, 240, respectively, during insertion of suture instrument and also inhibits barbs 230, 250 from catching, or snagging on tissue prior to or during insertion of suturing device 10. Elongated tubular member 100 includes a central lumen 118 defined therethrough. Central lumen 118 connects lumen 318 with aperture 214 such that suture 20 may be disposed through an entire length of suturing device 10. As shown in Fig. 6, flanges 229 and 249 are disposed within central aperture 344 of disc 340 when barb holders 220 and 240 are in the closed position.

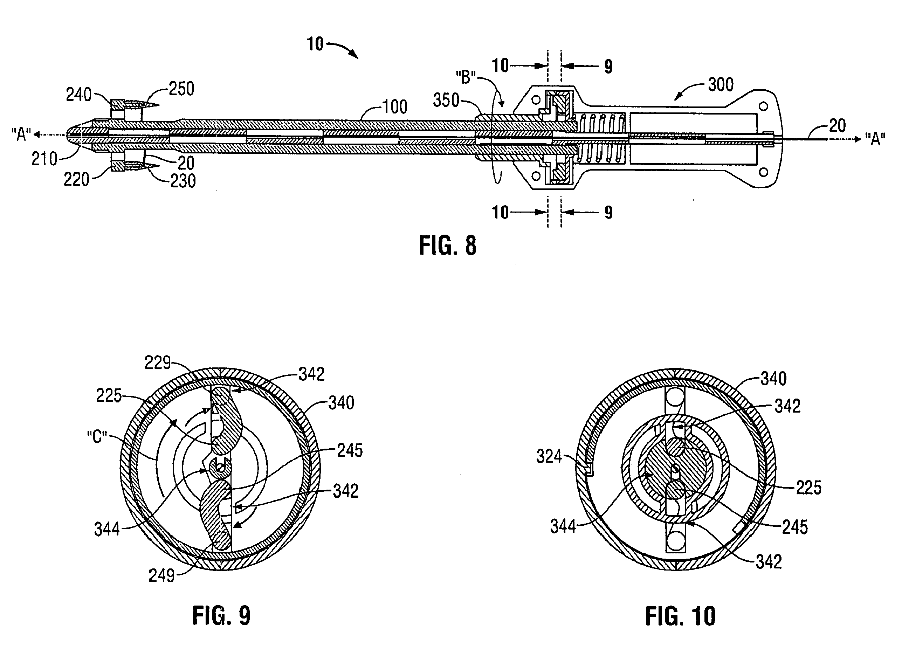

With reference now to Fig. 8-10, rotatable assembly 350 has been rotated in the direction of arrow "B" from the position shown in Figs. 5-7 such that barb holders 220 and 240 are in the open position, extending radially outwardly from the longitudinal axis "A." More specifically, the rotation of collar 350 rotates disc 340 and flanges 229 and 249 of rods 225 and 245 in the direction of arrow "C," respectively, such that the flanges 229 and 249 are disposed through cam slots 342 of disc 340. As mentioned above, the rotation of flanges 229 and 249 rotates rods 225 and 245, thereby rotating barb holders 220 and 240 to the position shown in Fig. 8. As shown in Fig. 10, a stop member 324 may be provided on disc 330 such that disc 340 may only be rotated to a specific position before contacting stop member 324, which inhibits further rotation. Stop member 324 may be configured to correspond to the maximum radially displaced position of barbs 330 and 350 with respect to longitudinal axis "A." This would help ensure that barbs 330 and 350 are rotated outwardly a sufficient distance but would help inhibit over-rotation of barbs 330, 350.

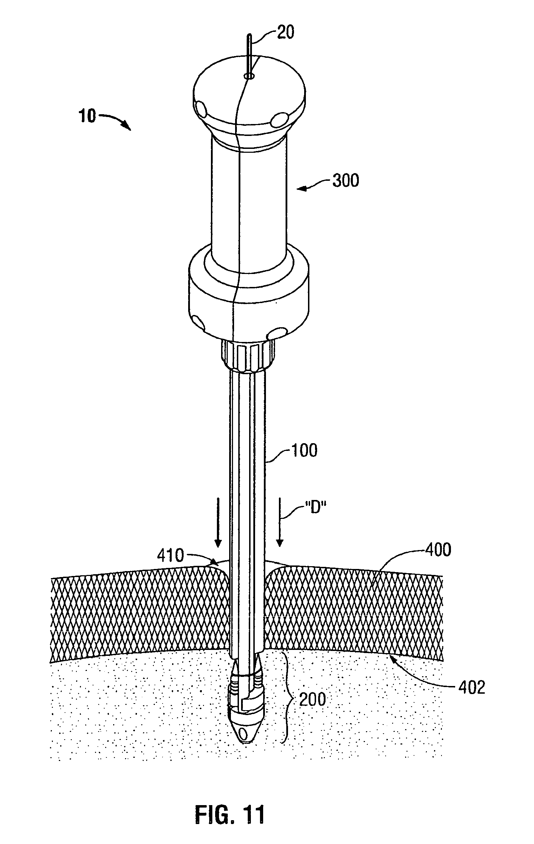

The operation of suturing device 10 will now be described with reference to Figs. 11-16. As shown in Fig. 11, suturing device 10 is initially in the closed position. In this position, instrument 10 is translated distally in the direction of arrow "D" through an opening 410 in tissue 400. Suturing device 10 is translated further distally until head assembly 200 is positioned proximally of an interior surface 402 of tissue 400, as shown in Fig. 11.

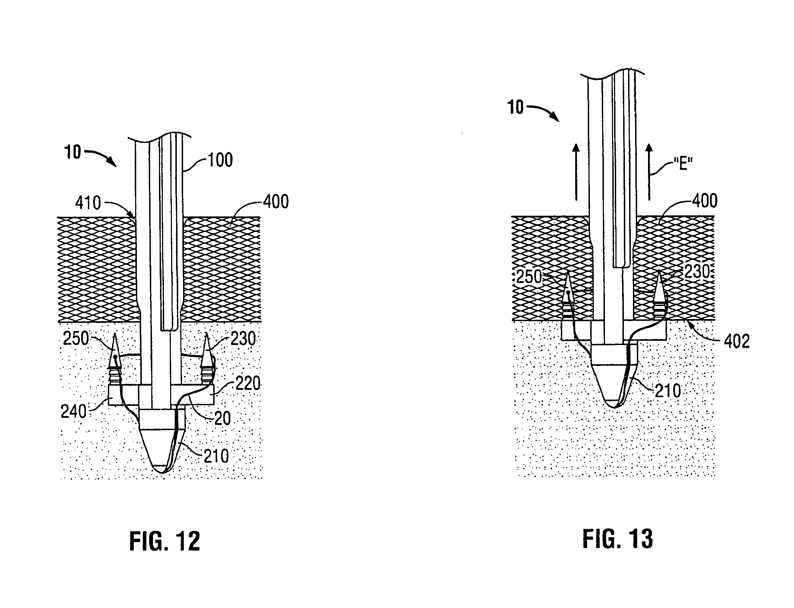

Referring now to Fig. 12, once suturing device 10 is positioned as described above, collar 350 is rotated, thereby rotating barb holders 220 and 240 outwardly from elongated tubular member 100 to the open position such that barbs 230, 250 are moved from an approximated, or adjacent position relative to elongated tubular member 100, to a spaced-apart position relative to elongated tubular member 100. It is envisioned that barb holders 220, 240 define a sufficient length such that, when moved to the spaced-apart, or open position, bars 230, 250 disposed on barb holders 220, 240, respectively, are positioned adjacent tissue 400 on opposing sides of opening 410 in tissue 400.

As shown in Fig. 12, barbs 230 and 250 are disposed on barb holders 220 and 240, respectively, each barb 230 and 250 retaining a portion of suture 20 therethrough. From this position, suturing device 10 is translated proximally in the direction of arrow "E," as shown in Fig. 13, thereby urging barbs 230 and 250 toward tissue such that barbs 230, 250 pierce through interior tissue surface 402, ultimately anchoring within tissue 400.

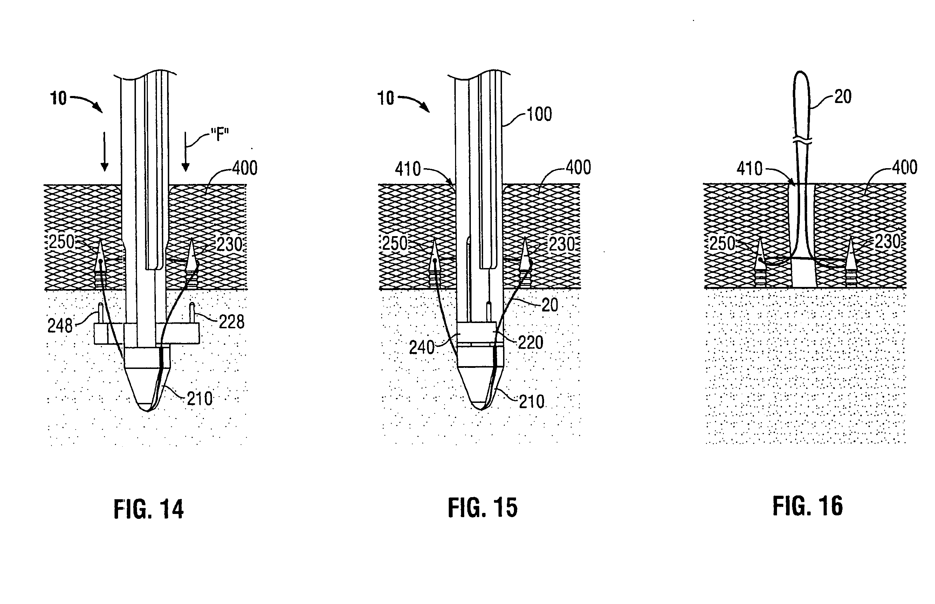

Once barbs 230 and 250 are anchored within tissue 400, as shown in Fig. 14, suturing device 10 may be translated distally to release barbs 230 and 250 from posts 228 and 248 of barb holders 220 and 240, respectively. Alternatively, a release mechanism (not shown) may be provided for selectively releasing barbs 230, 250 from barb holders 220, 240, respectively. Further, barbs 230, 250 may be engaged to barb holders 220, 240, respectively, via a frangible structure to permit release of barbs 230, 250 from barb holders 220, 240, respectively. In either embodiment, barbs 230, 250 are released from suturing device 10, leaving barbs 230 and 250, with portions of suture 20 disposed therethrough, in tissue 400.

Coiiar 350 (Fig. 2) may then be rotated in the reverse direction, i.e., collar 350 (Fig. 2) may be rotated back to its initial position, to rotate barb holders 220 and 240 back to the closed position, as shown in Fig. 15. From this position, suturing device 10 may be translated proximally out through opening 410 in tissue 400, leaving barbs 230 and 250 disposed through tissue 400 on either side of opening 410. Due to the configuration of suturing device 10, which includes a portion of suture 20 extending therethrough, suture 20 is left extending from barbs 230, 250 through opening 410 in tissue 400 upon removal of suturing device 10, allowing the surgeon to readily grasp suture 20. Suture 20 may then be tightened and tied off to close opening 410 in tissue 400, as shown in Fig. 16. More particularly, with a portion of suture 20 engaged to each of barbs 230, 250, tightening of suture 20 urges barbs 230, 250 toward one another, pulling the portions of tissue 400 on either side of opening 410 toward one another and, thus, closing opening 410. Once sufficiently tightened, i.e., once opening 410 is sufficiently closed, suture 20 may be tied off.

It is envisioned that barbs 230 and 250 may be formed from a bio-absorbable material such that they absorb into tissue 400, thereby obviating the need to remove barbs 230 and 250 after the procedure is complete. Suture 20 may also be formed from a bio-absorbable material for similar purposes. Suitable bio-absorbable materials include, but are not limited to polyglycolides, polylactides and copolymers thereof as well as natural materials (e.g. gut).

From the foregoing and with reference to the various figure drawings, those skilled in the art will appreciate that certain modifications can also be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.
The invention may be described by reference to the following numbered paragraphs-
1. A suturing device according to the present invention includes an elongated body and first and second rotatable barb holders operably coupled to the elongated body, each barb holder configured to releasably retain a barb thereon, each barb configured to retain a portion of a length of suture therethrough, the first and second rotatable barb holders being rotatable between a first position, wherein the barbs are positioned adjacent the elongated body, and a second position, wherein the barbs are radially spaced from the elongated body; for use in a method of suturing, wherein the method comprises the steps of providing a suturing device according to the present invention, moving the first and second rotatable barb holders to the first position; translating the suturing device proximally through an opening in tissue such that the barbs are positioned on an interior side of tissue; moving the first and second rotatable barb holders to the second position; translating the suturing device with respect to tissue to engage the barbs within tissue on either side of the opening in tissue; releasing the barbs from the barb holders; moving the first and second rotatable barb holders to the first position; and withdrawing the suturing device from the opening in tissue.
2. A suturing device according to paragraph 1, further comprising the steps of tying off the length of suture to close the opening in tissue.
3. A suturing device according to paragraph 1 or paragraph 2, wherein the suturing device is translated proximally with respect to tissue to engage the barbs within tissue on either side of the opening in tissue; and/or wherein the suturing device is translated distally to release the barbs from the rotatable barb holders once the barbs are engaged within tissue.
4. A suturing device according to any of paragraphs 1 to 3, wherein the suturing device further includes a head assembly disposed at a distal end thereof; and/or wherein the suturing device further includes a handle assembly disposed at a proximal end thereof.

## Claims

1. A suturing device, comprising:
an elongated tubular member defining a longitudinal axis, the elongated tubular member having a proximal portion and a distal portion; and
a head assembly extending from the distal portion of the elongated tubular member and disposed about the longitudinal axis, the head assembly including at least one rotatable barb holder coupled thereto, the at least one rotatable barb holder configured to releasably retain a barb thereon, the at least one rotatable barb holder being rotatable between a first position, wherein the barb is positioned adjacent the elongated tubular member, and a second position, wherein the barb is radially spaced from the elongated tubular member.

2. The suturing device according to claim 1, further comprising a handle assembly extending from the proximal portion of the elongated tubular member, at least a portion of the handle assembly being rotatable with respect to the elongated tubular member to rotate the at least one rotatable barb holder between the first position and the second position.

3. The suturing device according to claim 1 or claim 2, wherein the head assembly includes two rotatable barb holders, the barb holders being diametrically opposed with respect to the longitudinal axis when in the second position.

4. The suturing device according to any of claims 1 to 3, wherein the elongated tubular member is configured to house a length of suture therethrough.

5. The suturing device according to claim 4, wherein the head assembly is configured to allow passage of the length of suture therethrough.

6. The suturing device according to claim 5, wherein the length of suture extends from the head assembly and engages the barb.

7. The suturing device according to any preceding claim, wherein the barbs are formed from a bio-absorbable material; and/or wherein, in the first position, the barb is disposed within an outer radius of the elongated tubular member.

8. A suturing device, comprising:
an elongated tubular member defining a longitudinal axis, the elongated tubular member having a proximal portion and a distal portion and configured to house a length of suture therethrough; and
a head assembly extending from the distal portion of the elongated tubular member and disposed about the longitudinal axis, at least a portion of the head assembly configured to allow passage of the length of suture therethrough, the head assembly including at least one rotatable barb holder extending therefrom, the at least one rotatable barb holder configured for retaining a barb thereon and being rotatable between a first position and a second position, wherein the barb is disposed adjacent the elongated tubular member in the first position and wherein the barb is radially spaced from the elongated tubular member in the second position.

9. The suturing device according to claim 8, further comprising a rotatable collar disposed on the proximal portion of the elongated tubular member, the rotatable collar configured for rotating the at least one rotatable barb holder between the first position and the second position.

10. The suturing device according to claim 9, wherein each barb is adapted to retain a portion of the length of suture therethrough; and/or wherein the barbs are formed from bio-absorbable material; and/or wherein each barb holder is adapted to releasably accommodate at least one barb thereon.

11. The suturing device according to any preceding claim, wherein the barbs are retained on the barb holders in a generally proximally-facing orientation.

12. A suturing device according to any preceding claim including an elongated body and first and second rotatable barb holders operably coupled to the elongated body, each barb holder configured to releasably retain a barb thereon, each barb configured to retain a portion of a length of suture therethrough, the first and second rotatable barb holders being rotatable between a first position, wherein the barbs are positioned adjacent the elongated body, and a second position, wherein the barbs are radially spaced from the elongated body; for use in a method of suturing, wherein the method comprises the steps of:
providing a suturing device according to any preceding claim,
moving the first and second rotatable barb holders to the first position;
translating the suturing device proximally through an opening in tissue such that the barbs are positioned on an interior side of tissue;
moving the first and second rotatable barb holders to the second position;
translating the suturing device with respect to tissue to engage the barbs within tissue on either side of the opening in tissue;
releasing the barbs from the barb holders;
moving the first and second rotatable barb holders to the first position; and
withdrawing the suturing device from the opening in tissue.

13. A suturing device according to claim 12, further comprising the steps of tying off the length of suture to close the opening in tissue.

14. A suturing device according to claim 12 or claim 13, wherein the suturing device is translated proximally with respect to tissue to engage the barbs within tissue on either side of the opening in tissue; and/or wherein the suturing device is translated distally to release the barbs from the rotatable barb holders once the barbs are engaged within tissue.

15. A suturing device according to any of claims 12 to 14, wherein the suturing device further includes a head assembly disposed at a distal end thereof; and/or wherein the suturing device further includes a handle assembly disposed at a proximal end thereof.
